# EUROPEAN PATENT APPLICATION

(11) **EP 4 563 582 A1**
(43) Date of publication of application: **04.06.2025**
(21) Application number: 23846764.1
(22) Date of filing: 17.05.2023
(51) Int. Cl.: C07F 7/24, H10K 30/10

(54) **METHOD FOR SYNTHESIZING DELTA-PHASE PEROVSKITE CRYSTAL, AND DELTA-PHASE PEROVSKITE CRYSTAL PRODUCED THEREBY**

(30) Priority: 25.07.2022 KR 20220091609
(71) Applicant: Hanwha Solutions Corporation, Jung-gu Seoul 04541 (KR)
(72) Inventor: SHIN, Gwang Su, Seoul 04541 (KR); KIM, Song Hee, Seoul 04541 (KR); MA, Kyung Yeon, Seoul 04541 (KR)
(74) Representative: Mooser, Sebastian Thomas
(86) International application number: PCT/KR2023/006675
(87) International publication number: WO 2024/025103

(57) **Abstract**

The present invention relates to a method for producing a delta-phase perovskite crystal, and more specifically, to a novel method for synthesizing a delta-phase perovskite crystal at high yield and to a delta-phase perovskite crystal manufactured using same.

## Description

### [Technical Field]

The present invention relates to a method for synthesizing a delta-phase perovskite crystal with high yield and high purity, and to a delta-phase perovskite crystal manufactured using the same.

### [Background Art]

To solve the depletion of fossil fuels and the environmental problems caused by their use, research on renewable and clean alternative energy sources such as solar energy, wind power, and hydropower is actively being conducted.

Among these, interest in solar cells that directly convert the energy of sunlight into electrical energy is increasing significantly. Here, a solar cell is a cell that generates current-voltage by utilizing the photovoltaic effect in which light energy is absorbed from sunlight and electrons and holes are generated.

Currently, n-p diode-type silicon (Si) single crystal-based solar cells with a light energy conversion efficiency of over 20% can be manufactured and are used for actual solar power generation, and there are also solar cells using compound semiconductors such as gallium arsenide (GaAs) with even better conversion efficiencies. However, these inorganic semiconductor-based solar cells require materials refined with high purity to achieve high efficiency, and therefore much energy is consumed in refining the raw materials, and expensive processing equipment is required in the process of producing single crystals or thin films using the raw materials, which limits the reduction of the solar cell manufacturing cost and has been an obstacle to large-scale applications.

Accordingly, in order to manufacture solar cells at a low cost, it is necessary to drastically reduce the cost of materials or manufacturing processes that are essential to solar cells, and research is being conducted on perovskite solar cells that may be manufactured with low-cost materials and processes as an alternative to inorganic semiconductor-based solar cells.

The general structural formula of the perovskite structure is AMX₃, where the X position is occupied by anions, the A position is occupied by a large cation, and the M position is occupied by a small cation.

These perovskite compounds have excellent electrical conductivity, charge mobility, and optical properties, and therefore have a wide range of applications, and have various characteristics including long lifespan, high absorption wavelength spectra due to a small energy band gap, and wide charge-carrier diffusion length. At the same time, they have advantages in that they are economical in material price, can be produced with a solution, have low process costs, and can be manufactured at low temperatures, and are therefore drawing attention as promising materials for renewable energy applications. In particular, research is continuously being conducted on using them as light absorbers for perovskite solar cells.

The conventional synthetic method for manufacturing a perovskite crystal with the structure of ABX₃ (A = monovalent organic cation, B = divalent metal cation, X = halogen ion) involves synthesizing ABX₃ from AX and BX₂ at a high temperature in a solvent, followed by filtering and drying to obtain the product. However, since the synthetic yield is low, mass productivity is low, and unwanted by-products are generated during the synthesis of AX, which is a precursor used in the synthesis, and therefore there is a limitation that the yield is low.

### [Disclosure]

### [Technical Problem]

The present invention has been derived to overcome the above-described problems, and is directed to providing a method for manufacturing a delta-phase perovskite composite material with high yield and/or high purity as a method that is efficient and enables mass-production, using a different precursor from the conventional one, and a delta-phase perovskite composite material manufactured by the method.

### [Technical Solution]

To solve the above-described problems, the present invention relates to a method for manufacturing a delta-phase perovskite crystal, in which a process including: Step 1 of introducing a solvent into a reactor, and then introducing an organic anion precursor represented by Chemical Formula 1 below and a metal ion precursor represented by Chemical Formula 2 below into the solvent and dissolving the same to prepare a reaction solution; Step 2 of heating the reaction solution to 20 to 110 °C, and when the reaction solution turns yellow, introducing an anti-solvent to obtain a solution containing a precipitate; Step 3 of removing a colorless solution from the solution containing the precipitate of Step 2, and then performing primary washing of the precipitate with a bad solvent; Step 4 of performing secondary washing of the precipitate, which has undergone the primary washing, with an ether-based solvent, and filtering the same to obtain a filtrate; and Step 5 of drying the filtrate to obtain a delta-phase perovskite crystal represented by Chemical Formula 3 below is performed:

[Chemical Formula 1] AX;

[Chemical Formula 2] BX₂;

[Chemical Formula 3] ABX₃,

in the above Chemical Formulas 1 and 3, A is formamidinium (FA), methylammonium (MA), FAₓMA₍₁₋ₓ₎ (0<X<1), or N(R¹)₄⁺, and R¹ is a straight-chain alkyl group having 1 to 5 carbon atoms, a branched alkyl group having 3 to 5 carbon atoms, a phenyl group, an alkylphenyl group, or an alkoxyphenyl group, and in the above Chemical Formulas 2 and 3, B is Fe²⁺, Co²⁺, Ni²⁺, Cu²⁺, Sn²⁺, Pb²⁺, Bi²⁺, Ge²⁺, Ti²⁺, Eu²⁺, or Zr²⁺, and X is I⁻, Cl⁻, or Br⁻.

As one preferred embodiment of the present invention, the solvent in Step 1 may include one or more selected from γ-butyrolactone, γ-valerolactone, α-angelica lactone, α-methylene-γ-butyrolactone, α-hydroxy-γ-butyrolactone, and ε-caprolactone.

As one preferred embodiment of the present invention, in Step 1, the reaction solution may contain the organic anion precursor and the metal ion precursor in a molar ratio of 0.8 to 1.2:1.

As one preferred embodiment of the present invention, the anti-solvent may contain one or more selected from chlorobenzene, toluene, and xylene.

As one preferred embodiment of the present invention, the bad solvent may include a nitrile-based solvent including one or more selected from acetonitrile, propionitrile, and aminopropionitrile.

As one preferred embodiment of the present invention, the ether-based solvent may include one or more selected from diethyl ether, methyl tert-butyl ether, diisopropyl ether, and dibutyl ether.

As one preferred embodiment of the present invention, the delta-phase perovskite crystal obtained in Step 5 may have a yield higher than or equal to 75.00% and a purity higher than or equal to 99.00%, preferably a yield higher than or equal to 82.00% and a purity higher than or equal to 99.00%, and more preferably a yield higher than or equal to 90.00% and a purity higher than or equal to 99.00%.

Another object of the present invention is to provide a delta-phase perovskite crystal manufactured by the above-described method, and the perovskite composite material may be a perovskite crystal represented by Chemical Formula 3 below:

[Chemical Formula 3] ABX₃

in the above Chemical Formula 3, A is formamidinium (FA), methylammonium (MA), FAₓMA₍₁₋ₓ₎ (0<X<1), or N(R¹)₄⁺, R¹ is a straight-chain alkyl group having 1 to 5 carbon atoms, a branched alkyl group having 3 to 5 carbon atoms, a phenyl group, an alkylphenyl group, or an alkoxyphenyl group, B is Fe²⁺, Co²⁺, Ni²⁺, Cu²⁺, Sn²⁺, Pb²⁺, Bi²⁺, Ge²⁺, Ti²⁺, Eu²⁺, or Zr²⁺, and X is I⁻, Cl⁻, or Br⁻.

**In** addition, still another object of the present invention is to provide a perovskite solar cell including the perovskite crystal as a light absorption layer (or photoactive layer).

### [Advantageous Effects]

The present invention enables mass production of delta-phase perovskite crystals with high yield and high purity in an economical manner, and the delta-phase perovskite crystals of the present invention can be applied as materials in the fields of electricity, electronics, or optics requiring high electrical conductivity, charge mobility, and optical properties. As a preferred example, the delta-phase perovskite crystals can be applied as a light absorption layer (photoactive layer) of a solar cell.

### [Description of Drawings]

FIG. 1 shows photographs schematically representing the process of the delta-phase perovskite crystal synthesis process performed in Example 1.
FIG. 2 shows the X-ray diffraction (XRD) measurement results for each of the delta-phase perovskite crystals synthesized in Examples 1 to 5 and Comparative Examples 1 to 3, which were performed by introducing an anti-solvent under different temperature conditions.
FIG. 3 shows a thermogravimetric analysis (TGA) graph for the delta-phase perovskite crystal manufactured in Example 2.
FIG. 4 shows the performance test measurement results of the perovskite solar cells manufactured in Manufacturing Examples 1 to 3.

### [Modes of the Invention]

Hereinafter, the present invention will be described in more detail.

The present invention relates to a method of manufacturing a perovskite crystal represented by Chemical Formula 3 below.

[Chemical Formula 3] ABX₃

**In** Chemical Formula 3, A is formamidinium (FA), methylammonium (MA), FAₓMA₍₁₋ₓ₎ (0<X<1), or N(R¹)₄⁺, preferably FA, MA, or FAₓMA₍₁₋ₓ₎ (0<X<1), and more preferably FA or MA. In addition, R¹ is a straight-chain alkyl group having 1 to 5 carbon atoms, a branched alkyl group having 3 to 5 carbon atoms, a phenyl group, an alkylphenyl group, or an alkoxyphenyl group, and R¹ is preferably a straight-chain alkyl group having 1 to 5 carbon atoms, and more preferably a straight-chain alkyl group having 1 or 2 carbon atoms.

In addition, B in Chemical Formula 3 is Fe²⁺, Co²⁺, Ni²⁺, Cu²⁺, Sn²⁺, Pb²⁺, Bi²⁺, Ge²⁺, Ti²⁺, Eu²⁺, or Zr²⁺, preferably Cu²⁺, Sn²⁺, Pb²⁺, Bi²⁺, Ge²⁺, or Ti²⁺, and more preferably Sn²⁺, Pb²⁺, or Bi²⁺.

In addition, X in Chemical Formula 3 is I⁻, Cl⁻, or Br⁻, preferably I⁻ or Cl⁻, and more preferably I⁻.

Organic halides such as formamidinium iodide (FAI) are vulnerable to moisture, and purified organic halides are expensive. In addition, direct synthesis of organic halides during perovskite (ABX₃) synthesis is uneconomical in consideration of the low yield of the conventional perovskite synthesis method.

The delta-phase perovskite crystal of the present invention may be synthesized by performing a process including: Step 1 of introducing a solvent into a reactor, and then introducing an organic anion precursor represented by Chemical Formula 1 below and a metal ion precursor represented by Chemical Formula 2 below into the solvent and dissolving the same to prepare a reaction solution; Step 2 of heating the reaction solution to 20 to 110 °C, and when the reaction solution turns yellow, introducing an anti-solvent to obtain a solution containing a precipitate; Step 3 of removing a colorless solution from the solution containing the precipitate of Step 2, and then performing primary washing of the precipitate with a bad solvent; Step 4 of performing secondary washing of the precipitate, which has undergone the primary washing, with an ether-based solvent, and filtering the same to obtain a filtrate; and Step 5 of drying the filtrate to obtain a delta-phase perovskite crystal represented by Chemical Formula 3 below.

[Chemical Formula 1] AX

In Chemical Formula 1, A is FA, MA, FAₓMA₍₁₋ₓ₎ (0<X<1), or N(R¹)₄⁺, preferably FA, MA, or FAₓMA₍₁₋ₓ₎ (0<X<1), and more preferably FA or MA.

In addition, X of Chemical Formula 1 is I⁻, Cl⁻, or Br⁻, preferably I⁻ or Cl⁻, and more preferably I⁻.

[Chemical Formula 2] BX₂

B in Chemical Formula 2 is Fe²⁺, Co²⁺, Ni²⁺, Cu²⁺, Sn²⁺, Pb²⁺, Bi²⁺, Ge²⁺, Ti²⁺, Eu²⁺, or Zr²⁺, preferably Cu²⁺, Sn²⁺, Pb²⁺, Bi²⁺, Ge²⁺, or Ti²⁺, and more preferably Sn²⁺, Pb²⁺, or Bi²⁺. In addition, X in Chemical Formula 2 is I⁻, Cl⁻, or Br⁻, preferably I⁻or Cl⁻, and more preferably I⁻.

The solvent of Step 1 may include one or more selected from γ-butyrolactone, γ-valerolactone, α-angelica lactone, α-methylene-γ-butyrolactone, α-hydroxy-γ-butyrolactone, and ε-caprolactone, preferably one or more selected from γ-butyrolactone, γ-valerolactone, and α-angelica lactone, and more preferably one or more selected from γ-butyrolactone and γ-valerolactone.

In addition, the mixed solution of Step 1 may contain the organic anion precursor and the metal ion precursor in a molar ratio of 0.8 to 1.2:1, preferably the organic anion precursor and the metal ion precursor in a molar ratio of 0.9 to 1.2:1, and more preferably in a molar ratio of 0.95 to 1.1:1. At this time, when the molar ratio of the organic anion precursor is less than 0.8 or exceeds 1.2 based on the metal ion precursor, there may be a problem that the perovskite crystal yield is reduced.

Step 1 may be performed at 10 to 35 °C, preferably under room temperature conditions of 20 to 30 °C, and after introducing the organic anion precursor and the metal ion precursor to the solvent and stirring the resulting mixture for about one to two hours, the organic anion precursor and the metal ion precursor are completely dissolved in the solvent to form a transparent yellow reaction solution.

Next, in Step 2, the reaction solution formed in Step 1 in which the organic anion precursor and the metal ion precursor are dissolved is heated to 20 to 110 °C, preferably 50 to 100 °C, more preferably 50 to 80 °C, and even more preferably 55 to 70 °C, so that the reaction solution becomes opaque or dark yellow.

Then, when an anti-solvent is added dropwise, a yellow precipitate is generated, and the anti-solvent is added dropwise until the solution becomes colorless or transparent. At this time, a benzene-based anti-solvent is preferably used rather than an ether-based solvent such as diethyl ether, methyl tert-butyl ether, or dimethyl ether, which has a low yield of about 30%.

At this time, when the temperature is higher than 110 °C, the yield of the delta-phase perovskite crystal is too low due to the increase in an alpha-phase perovskite crystal, and when it is lower than 20 °C, the usage amount of an anti-solvent becomes too much, the reaction time becomes long, and the yield of the delta-phase perovskite crystal decreases, which is uneconomical.

In addition, the input amount of an anti-solvent is appropriately about 200 to 400:100 by volume, preferably about 230 to 370:100 by volume, and more preferably about 260 to 340:100 by volume, based on the total volume of the solvent used in Step 1.

In addition, the anti-solvent may include one or more selected from chlorobenzene, toluene, and xylene, preferably one or more selected from chlorobenzene and toluene, and more preferably toluene.

In addition, a drying process for removing the reaction solvent remaining in the precipitate obtained in Step 2 before performing Step 3 described below may be omitted.

Next, Step 3 is a process of removing a transparent and/or colorless solution and then performing primary washing of the precipitate with a bad solvent. As the bad solvent, it is preferable to use a nitrile-based solvent that has a low boiling point and high volatility, does not dissolve the perovskite crystal, which is the reaction product, but well dissolves organic anion precursors, which are the reaction residues, and has good miscibility with the solvent of Step 1.

As the nitrile-based solvent, one or more selected from acetonitrile, propionitrile, and aminopropionitrile may be used, and preferably, one or more selected from acetonitrile and propionitrile may be used.

In addition, the primary washing of Step 3 may be repeatedly performed one to three times, preferably two to three times.

Next, Step 4 is a process of removing the bad solvent used in the washing in Step 3, performing secondary washing with an ether-based solvent, and then filtering to obtain a filtrate, and the secondary washing is performed to remove the solvent used in Step 1 that may remain and the bad solvent used in the primary washing.

The ether-based solvent used in the secondary washing may include at least one selected from diethyl ether, methyl *tert*-butyl ether, diisopropyl ether, and dibutyl ether, preferably one or more selected from diethyl ether, methyl tert-butyl ether, and dibutyl ether, and more preferably one or more selected from diethyl ether and methyl *tert-*butyl ether.

In addition, the filtering may be performed through a general filtration method used in the art, and preferably, the filtering may be performed by performing vacuum filtration.

In addition, the washing and filtering in Step 4 are preferably performed one or more times, preferably two to five times, and preferably three to five times, in order to improve the purity of the delta-phase perovskite crystal.

Next, Step 5 is a process of drying the filtrate to obtain a yellow delta-phase perovskite crystal represented by Chemical Formula 3, and the drying may be performed by a general method used in the art, and as a preferred example, the filtrate obtained in Step 4 may be stored in a vacuum oven for 12 to 36 hours to completely dry the same.

The above-described delta-phase perovskite crystal obtained through synthesis according to the method of the present invention may have a yield higher than or equal to 75.00%, preferably higher than or equal to 82.00%, more preferably higher than or equal to 90.00%, and even more preferably 93.0% to 99.0%, when measured according to Equation 1 below. Yield (%) = (Weight of obtained delta-phase perovskite crystal)/(Weight of organic anion precursor used in reaction + weight of metal ion precursor used in reaction) × 100%

The delta-phase perovskite crystal synthesized and obtained by the above-described method of the present invention may have a purity of 99.00% or higher.

The delta-phase perovskite crystal manufactured by the above-described method can be applied as a material in the fields of electricity, electronics, or optics (solar cells, displays, lasers, sensors, etc.) that require high electrical conductivity, charge mobility, and optical properties. As a preferable example, the delta-phase perovskite crystal can be used as a precursor to coat a perovskite layer that can be used as a light absorption layer (photoactive layer) of a solar cell, and the light absorption layer having a target band gap can be controlled more easily. Therefore, the band gap can be efficiently controlled in an indirect manner.

A preferred example of a perovskite solar cell using a delta-phase perovskite crystal manufactured by the method of the present invention as a light absorption layer will be described below.

The perovskite solar cell may be a pin-structured perovskite solar cell, an inverted-structured perovskite solar cell, a tandem perovskite solar cell, or a tandem silicon/perovskite heterojunction solar cell.

A preferred embodiment of a perovskite solar cell is a solar cell including a laminate having a structure in which a hole transport layer (HTL), a perovskite light absorption layer, an electron transporting layer (ETL), a conductive passivation layer, and a source electrode are sequentially laminated.

In addition, when the solar cell is an inverted-structure perovskite solar cell, the solar cell may have a form in which the laminate is laminated on top of a drain electrode.

In addition, the inverted-structure perovskite solar cell may be formed by sequentially laminating a conductive substrate, a drain electrode, an HTL, a light absorption layer, an ETL, and a source electrode as one set, and stacking the set as a single layer or multiple layers.

As another preferred embodiment, when the solar cell of the present invention is a tandem silicon/perovskite heterojunction solar cell, the solar cell may have a form in which a drain electrode, a silicon solar cell, a recombination layer, and the laminate are sequentially laminated.

The laminate constituting the perovskite solar cell will now be described in detail through the method of manufacturing the same. The laminate may be manufactured by performing a process including: Step 1 of forming an ETL by applying a coating agent for forming an ETL on top of the perovskite light absorption layer of the laminate including an HTL and a perovskite light absorption layer; Step 2 of forming a passivation layer on the ETL through a deposition process; and Step 3 of forming a source electrode on top of the passivation layer.

The HTL may include an inorganic and/or organic hole transport material. The inorganic hole transport material may include one or more selected from a nickel oxide (NiOₓ), CuSCN, CuCrO₂, and CuI.

The organic hole transport material may include a carbazole derivative, a polyarylalkane derivative, a phenylenediamine derivative, an arylamine derivative, an amino-substituted chalcone derivative, a styrylanthracene derivative, a fluorene derivative, a hydrazone derivative, a stilbene derivative, a silazane derivative, an aromatic tertiary amine compound, a styrylamine compound, an aromatic dimethylidine compound, a porphyrin compound, a phthalocyanine compound, a polythiophene derivative, a polypyrrole derivative, a polyparaphenylenevinylene derivative, pentacene, coumarin 6 (3-(2-benzothiazolyl)-7-(diethylamino)coumarin), zinc phthalocyanine (ZnPC), copper phthalocyanine (CuPC), titanium oxide phthalocyanine (TiOPC), 2,2',7,7'-tetrakis(N,N-p-dimethoxyphenylamino)-9,9'-spirobifluorene (spiro-MeOTAD), copper(II) 1,2,3,4,8,9,10,11,15,16,17,18,22,23,24,25-hexadecafluoro-29H,31H-phthalocyanine (F16CuPC), boron subphthalocyanine chloride (SubPc), N3(cis-di(thiocyanato)-bis(2,2'-bipyridyl-4,4'-dicarboxylic acid)-ruthenium(II), poly[3-hexylthiophene] (P3HT), poly[2-methoxy-5-(3',7'-dimethyloctyloxyl)]-1,4-phenylene vinylene (MDMO-PPV), poly[2-methoxy-5-(2''-ethylhexyloxy)-p-phenylene vinylene] (MEH-PPV), poly(3-octyl thiophene) (P3OT), poly(octyl thiophene) (POT), poly(3-decyl thiophene) (P3DT), poly(3-dodecyl thiophene) (P3DDT), poly(p-phenylene vinylene) (PPV), poly(9,9'-dioctylfluorene-co-N-(4-butylphenyl)diphenyl amine (TFB), polyaniline, [2,22',7,77'-tetrakis (N,N-di-pmethoxyphenyl amine)-9,9,9'-spirobi fluorine] (Spiro-MeOTAD), CuSCN, CuI, poly[2,1,3-benzothiadiazole-4,7-diyl[4,4-bis(2-ethylhexyl-4H-cyclopenta [2,1-b:3,4-b']dithiophene-2,6-diyl]] (PCPDTBT), (poly [(4,4'-bis(2-ethylhexyl)dithieno[3,2-b:2',3'-d]silole)-2,6-diyl-alt-(2,1,3-benzothiadiazole)-4,7-diyl]) (Si-PCPDTBT), poly(4,8-diethylhexyloxyl) (PBDTTPD), poly[2,7-(9-(2-ethylhexyl)-9-hexyl-fluorene)-alt-5,5-(4',7,-di-2-thienyl-2',1',3'-benzothiadiazole)] (PFDTBT), poly[2,7-.9,9-(dioctyl-fluorene)-alt-5,5-(4',7'-di-2-.thienyl-2',1',3'-benzothiadiazole)] (PFO-DBT), poly[(2,7-dioctylsilafluorene)-2,7-diyl-alt-(4,7-bis(2-thienyl)-2,1,3-benzothiadiazole)-5,5'-diyl]), PCDTBT(poly[[9-(1-octylnonyl)-9H-carbazole-2,7-diyl] - 2,5-thiophenediyl-2,1,3-benzothiadiazole-4,7-diyl-2,5-thiophenediyl] (PSiFDTBT), poly(9,9'-dioctylfluorene-co-bis(N,N'-(4,butylphenyl))bis(N,N'-phenyl-1,4-phenylene)diamine (PFB), poly(9,9'-dioctylfluorene-cobenzothiadiazole) (F8BT), poly(3,4-ethylenedioxythiophene) (PEDOT), poly(3,4-ethylenedioxythiophene) poly(styrenesulfonate) (PEDOT:PSS), poly(triarylamine) (PTAA), [2-(9H-carbazol-9-yl)ethyl]phosphonic acid (2-PACz), and/or [2-(3,6-dimethoxy-9H-carbazol-9-yl)ethyl]phosphonic acid (MeO-2PACz).

In addition, examples of the method of forming the HTL may include a coating method and a vacuum deposition method, and examples of the coating method may include a gravure coating method, a bar coating method, a printing method, a spray method, a spin coating method, a dip coating method, and a die coating method.

Next, the light absorption layer may include the above-described delta-phase perovskite crystal represented by Chemical Formula 3.

Next, the ETL may include an inorganic material (e.g., a metal oxide) and/or an organic material. The ETL may be a flat metal oxide layer, a metal oxide layer having a surface roughness, a metal oxide layer of a composite structure in which a nanostructure (including metal oxide particles, nanowires, and/or nanotubes) of a homogeneous or heterogeneous metal oxide is formed on the surface of a metal oxide in a thin film shape, or a porous metal oxide layer, and preferably, the ETL may include a compact metal oxide layer and a meso-porous metal oxide layer, and preferable examples may include TiO₂, SnO₂, ZnO, and the like. In addition, the ETL may include phenyl-C61-butyric acid methyl ester (PCBM) as the organic material.

In addition, among the solar cell components, the upper electrode (or source electrode) may be formed by applying or depositing one or more materials selected from Pt, Au, Ni, Cu, Ag, In, Ru, Pd, Rh, Ir, Os, C, and a conductive polymer.

In addition, the solar cell may further include a passivation layer between the light absorption layer and the ETL.

Hereinafter, the present invention will be described more specifically through examples. However, the following examples do not limit the scope of the present invention, and should be interpreted as helping to understand the present invention.

### [Examples]

### Example 1: Synthesis of delta-phase perovskite crystal

After introducing γ-butyrolactone, which is a lactone-based solvent, to an Erlenmeyer flask, an organic anion precursor represented by Chemical Formula 1-1 below and a metal ion precursor represented by Chemical Formula 2-1 below were introduced and dissolved to prepare a transparent yellow reaction solution.

At this time, the organic anion precursor and the metal ion precursor were mixed in a molar ratio of 1:1.

Next, the reaction solution was heated to about 25 °C, the reaction solution became turbid and turned into an opaque yellow color, and toluene, which is an anti-solvent, was added dropwise to the reaction solution while stirring.

A precipitate was generated by the addition of the anti-solvent, and the anti-solvent toluene was added dropwise until the reaction solution became colorless (or transparent). At this time, the input amount of the anti-solvent was about 3 times the volume of the usage amount of the γ-butyrolactone.

Next, after removing the colorless (or transparent) reaction solution, a bad solvent acetonitrile was introduced to the precipitate, after which the solvent turned cloudy. Washing was repeated three times by adding the solvent until it became transparent and then discarding and removing the solvent.

Next, the precipitate that had undergone the primary washing was washed with diethyl ether and filtered through vacuum filtration to obtain a filtrate, and the washing and filtering were repeated twice.

Next, the filtrate was stored in a vacuum oven for 24 hours to completely dry the filtrate, thereby obtaining a delta-phase perovskite crystal represented by Chemical Formula 3-1 below (yield: 91.2%, purity: 99.90% to 99.95%).

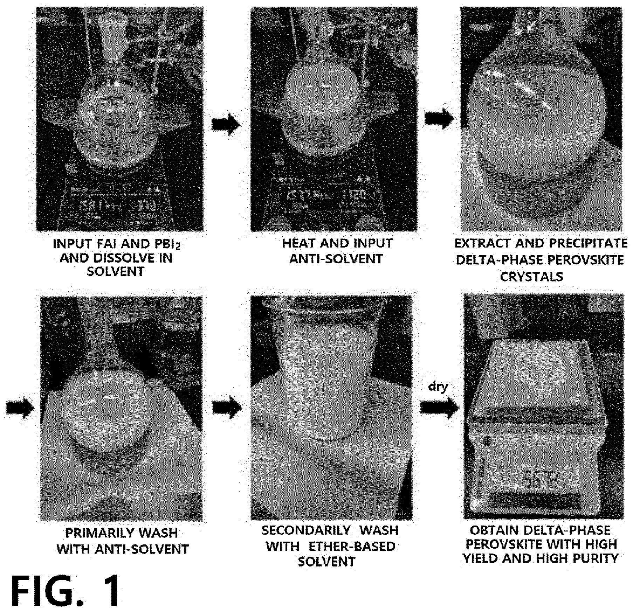

In addition, the manufacturing process photographs are shown in FIG. 1.

[Chemical Formula 1-1] AX

[Chemical Formula 2-1] BX₂

[Chemical Formula 3-1] ABX₃

In Chemical Formulas 1-1 and 3-1, A is FA, and in Chemical Formulas 1-1, 2-1, and 3-1, B is Pb²⁺, and X is I⁻.

### Examples 2 and 3 and Comparative Examples 1 to 3

Examples 2 and 3 and Comparative Examples 1 to 3 were performed by preparing perovskite crystals using the same method as in Example 1, but when toluene, which is an anti-solvent, was introduced to the reaction solution, the temperature of the reaction solution was varied as shown in Table 1 below to prepare each of the perovskite crystals.

### Example 4

A perovskite crystal was prepared using the same method as in Example 2, but chlorobenzene was used instead of toluene as an anti-solvent to prepare a delta-phase perovskite crystal.

### Example 5

A perovskite crystal was prepared using the same method as in Example 2, but xylene was used instead of toluene as an anti-solvent to prepare a delta-phase perovskite crystal.

### Comparative Example 4

A perovskite crystal was prepared using the same method as in Example 1, but diethyl ether, which is an ether-based solvent, was used instead of toluene as an anti-solvent to prepare a delta-phase perovskite crystal.

**[Table 1]**

| Classification | Temperature | Molar ratio of organic anionic precursor to metal ion precursor | Anti-solvent | Bad solvent |
|---|---|---|---|---|
| Example 1 | 25 °C | 1:1 | Toluene | Acetonitrile |
| Example 2 | 60 °C | 1:1 | Toluene | Acetonitrile |
| Example 3 | 90 °C | 1:1 | Toluene | Acetonitrile |
| Example 4 | 60 °C | 1:1 | Chlorobenzene | Acetonitrile |
| Example 5 | 60 °C | 1:1 | Xylene | Acetonitrile |
| Comparative Example 1 | 120 °C | 1:1 | Toluene | Acetonitrile |
| Comparative Example 2 | 150 °C | 1:1 | Toluene | Acetonitrile |
| Comparative Example 3 | 180 °C | 1:1 | Toluene | Acetonitrile |
| Comparative Example 4 | 25 °C | 1:1 | Diethyl ether | Acetonitrile |

### Experimental Example 1: X-ray diffraction (XRD) measurement and thermogravimetric analysis (TGA) measurement

### (1) XRD measurement

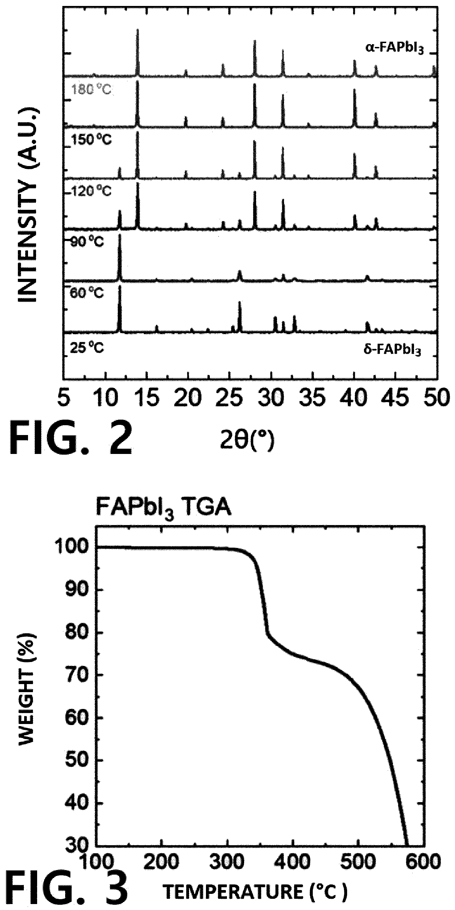

FIG. 2 shows the XRD measurement results of the delta-phase perovskite crystals manufactured in Examples 1 to 3 and Comparative Examples 1 to 3.

Referring to FIG. 2, it can be confirmed that the obtained (synthesized) phase of the delta-phase perovskite crystal (powder) represented by Chemical Formula 3-1 changed as the temperature of the solvent (γ-butyrolactone) in which the organic anion precursor and the metal ion precursor were dissolved was changed.

At a temperature lower than or equal to 90 °C, almost all of the synthesized perovskite crystals can be obtained in the delta phase, and in the range of about 110 to 150 °C, the alpha phase and the delta phase are obtained as a mixture. In addition, it can be confirmed that in the range of 150 to 180 °C, most of the synthesized perovskite crystals are obtained in the alpha phase.

### (2) TGA measurement

The TGA measurement results of the delta-phase perovskite crystal manufactured in Example 2 are shown in FIG. 3, and it was confirmed that the manufactured crystal was a high-purity delta-phase perovskite crystal.

### Experimental Example 2: Yield measurement

The synthesis yield of the delta-phase perovskites manufactured in Examples 1 to 5 and Comparative Examples 1 to 4 was measured according to Equation 1 below, and the results are shown in Table 2 below.

In addition, the XRD measurement results according to the temperature are shown in FIG 2. Yield of delta-phase perovskite (%) = (Weight of obtained delta-phase perovskite crystal)/(Weight of organic anion precursor used in reaction + weight of metal ion precursor used in reaction) × 100%

**[Table 2]**

| Classification | Temperature | Anti-solvent | Total yield of perovskite (%)⁽¹⁾ | Yield of delta-phase perovskite in total yield (%) |
|---|---|---|---|---|
| Example 1 | 25 °C | Toluene | 91.2 | 91.2 |
| Example 2 | 60 °C | Toluene | 97.3 | 97.3 |
| Example 3 | 90 °C | Toluene | 90.8 | 90.8 |
| Example 4 | 60 °C | Chlorobenzene | 75.3 | 75.3 |
| Example 5 | 60 °C | Xylene | 82.4 | 82.4 |
| Comparative Example 1 | 120 °C | Toluene | 91.1 | 36.2 |
| Comparative Example 2 | 150 °C | Toluene | 93.3 | 1.1 |
| Comparative Example 3 | 180 °C | Toluene | 94.4 | 0 |
| Comparative Example 4 | 25 °C | Diethyl ether | 40.4 | 37.0 |
| (1) The total yield of perovskite (%) refers to the total perovskite yield including the delta-phase and the alpha-phase. | | | | |

Referring to Table 2, in the case of Examples 1 to 5 where the perovskite was synthesized using an organic anion precursor and a metal ion precursor at a temperature lower than or equal to 90 °C, the delta-phase perovskite accounted for most of the total obtained perovskite yield. In addition, in the case of Example 4 where chlorobenzene was used as an anti-solvent and Example 5 where xylene was used, the total perovskite yield itself tended to be somewhat lower compared to Example 2 where toluene was used as an anti-solvent.

In addition, the total perovskite yield itself was higher in Example 2 where the synthesis was performed at 60 °C than in Example 1 where the synthesis was performed at 25 °C, and the total perovskite yield was lower in Example 3 where the synthesis was performed at 90 °C than in Example 2.

In addition, in the case of Comparative Examples 1 to 3, where the perovskite was synthesized using an organic anion precursor and a metal ion precursor at 120 to 180 °C, the total perovskite yield was high, but Comparative Example 1 exhibited a significantly lower delta-phase perovskite yield compared to Example 3, and mostly the alpha-phase perovskite was obtained in Comparative Examples 2 and 3.

In addition, in the case of Comparative Example 4, where diethyl ether was used as an anti-solvent, the total perovskite yield itself was low compared to Example 2, and the delta-phase yield was also relatively low.

Through these results, it was confirmed that the range of 40 to 90 °C, preferably 50 to 80 °C, was optimal for the organic anion precursor and the metal ion precursor in terms of the total perovskite yield and delta-phase perovskite yield, productivity, and economic efficiency.

### Manufacturing Example 1: Manufacturing of perovskite solar cell

The delta-phase perovskite crystal manufactured in Example 2, CsBr, and PbBr₂ were dissolved in dimethylformamide (DMF) and N-methyl-2-pyrrolidinone (NMP) to prepare a coating agent for forming a perovskite thin film, and then a single element of an opaque perovskite solar cell represented by (FAPbI₃)_{0.8}(CsPbBr₃)_{0.2} was manufactured using the same (active area: 0.096 cm²). Specifically, the structure of the opaque single element included NiOx (17 nm), 2PACz (1 nm), a perovskite thin film ((FAPbI₃)_{0.8}(CsPbBr₃)_{0.2}, 550 nm), LiF (1 nm), C60 (13 nm), BCP (8 nm), and a Ag electrode (200 nm) sequentially laminated on an indium tin oxide (ITO, 100 nm)-deposited organic substrate.

### Manufacturing Examples 2 and 3

Manufacturing Examples 2 and 3 were performed by manufacturing perovskite solar cells using the same method as Manufacturing Example 1, but the perovskite crystals manufactured in Examples 4 and 5, instead of Example 2, were used to manufacture the solar cells, respectively.

### Comparative Manufacturing Example 1

FAI, PbI₂, CsBr, and PbBr₂ were dissolved in DMF and NMP to prepare a coating agent (precursor solution) for forming a perovskite thin film, and then a single element of an opaque perovskite solar cell represented by (FAPbI₃)_{0.8}(CsPbBr₃)_{0.2} was manufactured using the same (active area: 0.096 cm²). Specifically, the structure of the opaque single element included NiOx (17 nm), 2PACz (1 nm), a perovskite thin film ((FAPbI₃)_{0.8}(CsPbBr₃)_{0.2}, 550 nm), LiF (1 nm), C60 (13 nm), BCP (8 nm), and a Ag electrode (200 nm) sequentially laminated on an indium tin oxide (ITO, 100 nm)-deposited organic substrate.

### Experimental Example 2: Performance measurement of solar cell elements

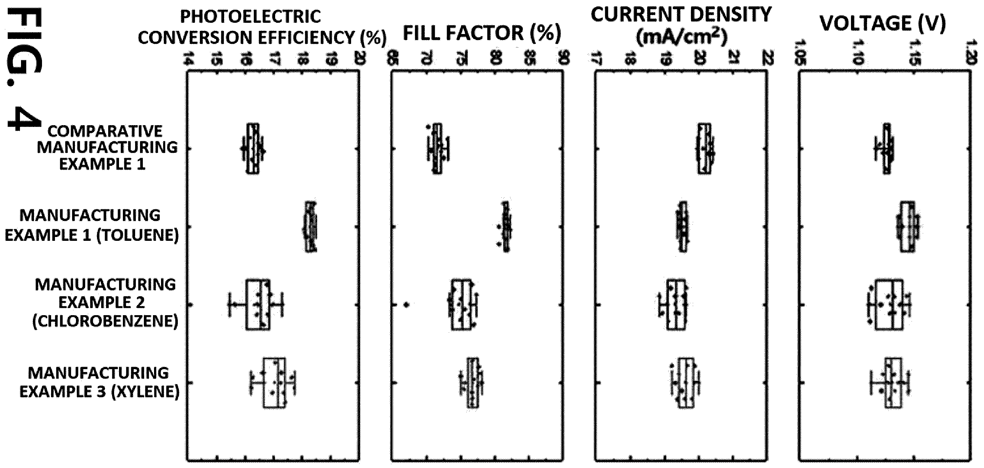

The current-voltage characteristics and efficiency of the solar cells manufactured in Manufacturing Examples 1 to 3 were measured, and the results are shown in Table 3 and FIG. 4 below.

**[Table 3]**

| Classification | Perovskite | Open circuit voltage (*V_{oc}*, V) | Short circuit current density (*J_{sc},* mA/cm²) | Fill factor (FF, %) | Photoelectric conversion efficiency (PCE, %) |
|---|---|---|---|---|---|
| Comparative Manufacturing Example 1 | FAI + PbI₂ | 1.126 | 20.21 | 71.63 | 16.31 |
| Manufacturing Example 1 | Example 2 | 1.145 | 19.54 | 81.74 | 18.30 |
| Manufacturing Example 2 | Example 4 | 1.129 | 19.31 | 74.84 | 16.33 |
| Manufacturing Example 3 | Example 5 | 1.131 | 19.61 | 76.81 | 17.05 |

Referring to Table 3 and FIG. 4, it can be confirmed that all of Manufacturing Examples 1 to 3 have relatively higher photoelectric conversion efficiencies than Comparative Manufacturing Example 1, and in particular, Manufacturing Example 1 had a photoelectric conversion efficiency that was about 2% higher than that of Comparative Manufacturing Example 1.

Through the above-described examples and experimental examples, it was confirmed that delta-phase perovskite crystals can be manufactured with a high yield while using a smaller amount of chemicals than conventional synthesis methods, and it was confirmed that when delta-phase perovskite crystals are applied as a light absorption layer material, a perovskite solar cell with excellent efficiency can be provided.

## Claims

1. A method of manufacturing a delta-phase perovskite crystal, the method comprising:
Step 1 of introducing a solvent into a reactor, and then introducing an organic anion precursor represented by Chemical Formula 1 below and a metal ion precursor represented by Chemical Formula 2 below into the solvent and dissolving the same to prepare a reaction solution;
Step 2 of heating the reaction solution to 20 to 110 °C, and when the reaction solution turns yellow, introducing an anti-solvent to obtain a solution containing a precipitate;
Step 3 of removing a colorless solution from the solution containing the precipitate of Step 2, and then performing primary washing of the precipitate with a bad solvent;
Step 4 of performing secondary washing of the precipitate, which has undergone the primary washing, with an ether-based solvent, and filtering the same to obtain a filtrate; and
Step 5 of drying the filtrate to obtain a delta-phase perovskite crystal represented by Chemical Formula 3 below:
[Chemical Formula 1] AX;
[Chemical Formula 2] BX₂;
[Chemical Formula 3] ABX₃,
in the above Chemical Formulas 1 and 3, A is formamidinium (FA), methylammonium (MA), FAₓMA₍₁₋ₓ₎ (0<X<1), or N(R¹)₄⁺, and R¹ is a straight-chain alkyl group having 1 to 5 carbon atoms, a branched alkyl group having 3 to 5 carbon atoms, a phenyl group, an alkylphenyl group, or an alkoxyphenyl group, and
in the above Chemical Formulas 2 and 3, B is Fe²⁺, Co²⁺, Ni²⁺, Cu²⁺, Sn²⁺, Pb²⁺, Bi²⁺, Ge²⁺, Ti²⁺, Eu²⁺, or Zr²⁺, and X is I⁻, Cl⁻, or Br⁻.

2. The method of claim 1, wherein the solvent in Step 1 includes one or more selected from γ-butyrolactone, γ-valerolactone, α-angelica lactone, α-methylene-γ-butyrolactone, α-hydroxy-γ-butyrolactone, and ε-caprolactone.

3. The method of claim 1, wherein, in Step 1, the reaction solution contains the organic anion precursor and the metal ion precursor in a molar ratio of 0.8 to 1.2:1.

4. The method of claim 1, wherein the anti-solvent contains one or more selected from chlorobenzene, toluene, and xylene.

5. The method of claim 1, wherein the input amount of the anti-solvent of Step 2 is 200 to 400: 100 by volume based on the total volume of the solvent used in Step 1.

6. The method of claim 1, wherein the bad solvent includes a nitrile-based solvent including one or more selected from acetonitrile, propionitrile, and aminopropionitrile, and the ether-based solvent includes one or more selected from diethyl ether, methyl tert-butyl ether, diisopropyl ether, and dibutyl ether.

7. The method of claim 1, wherein the delta-phase perovskite crystal obtained in Step 5 has a yield higher than or equal to 75% and a purity higher than or equal to 99%.

8. A delta-phase perovskite crystal manufactured by the method of any one selected from claims 1 to 7,
wherein the delta-phase perovskite crystal is a perovskite crystal represented by Chemical Formula 3 below:
[Chemical Formula 3] ABX₃
in the above Chemical Formula 3, A is formamidinium (FA), methylammonium (MA), FAₓMA₍₁₋ₓ₎ (0<X<1), or N(R¹)₄⁺, R¹ is a straight-chain alkyl group having 1 to 5 carbon atoms, a branched alkyl group having 3 to 5 carbon atoms, a phenyl group, an alkylphenyl group, or an alkoxyphenyl group, B is Fe²⁺, Co²⁺, Ni²⁺, Cu²⁺, Sn²⁺, Pb²⁺, Bi²⁺, Ge²⁺, Ti²⁺, Eu²⁺, or Zr²⁺, and X is I⁻, Cl⁻, or Br⁻.
